# EUROPÄISCHE PATENTSCHRIFT

(11) **EP 2 117 467 B1**
(45) Veröffentlichungstag und Bekanntmachung des Hinweises auf die Patenterteilung: **27.07.2016**
(21) Anmeldenummer: 08709075.9
(22) Anmeldetag: 18.02.2008
(51) Int. Cl.: A61B 5/00, A61B 5/04, A61B 3/113, A61F 2/16, G01R 33/07

(54) **IMPLANTIERBARES SYSTEM ZUR BESTIMMUNG DES AKKOMMODATIONSBEDARFS DURCH MESSUNG DER AUGAPFELORIENTIERUNG UNTER NUTZUNG EINES EXTERNEN MAGNETFELDS**
IMPLANTABLE SYSTEM FOR DETERMINING THE ACCOMMODATION REQUIREMENT BY MEASURING THE EYEBALL ORIENTATION USING AN EXTERNAL MAGNETIC FIELD
SYSTÈME IMPLANTABLE POUR DÉTERMINER LE BESOIN D'ACCOMMODATION PAR MESURE DE L'ORIENTATION DU GLOBE OCCULAIRE AU MOYEN D'UN CHAMP MAGNÉTIQUE EXTERNE

(30) Priorität: 21.02.2007 DE 102007008374
(43) Veröffentlichungstag der Anmeldung: 18.11.2009
(73) Patentinhaber: Karlsruher Institut für Technologie, 76131 Karlsruhe (DE); Universität Rostock, 18051 Rostock (DE)
(72) Erfinder: KLINK, Simon, 76329 Stuttgart (DE); BRETTHAUER, Georg, 76139 Karlsruhe (DE); GUTHOFF, Rudolf, 18119 Rostock (DE); GENGENBACH, Ulrich, 75196 Remchingen (DE); BERGEMANN, Mark, 73033 Göppingen (DE); KOKER, Torsten, 76297 Stutensee (DE); RÜCKERT, Wolfgang, 42489 Wülfrath (DE)
(74) Vertreter: Fitzner, Uwe
(86) Internationale Anmeldenummer: PCT/EP2008/051938
(87) Internationale Veröffentlichungsnummer: WO 2008/101898

(56) Entgegenhaltungen:
- WO-A-2006/034336
- FR-A- 2 833 477
- US-A1- 2005 256 571
- RODRÍGUEZ F ET AL: "Eye-movement recording in freely moving animals" PHYSIOLOGY AND BEHAVIOR, ELSEVIER SCIENCE LTD., OXFORD, GB, Bd. 72, Nr. 4, 1. März 2001 (2001-03-01), Seiten 455-460, XP002363772 ISSN: 0031-9384

## Beschreibung

Die vorliegende Anmeldung nimmt die Priorität der 10 2007 008 374.4-55 in Anspruch.

Gegenstand der Erfindung ist ein implantierbares System zur Bestimmung des Akkommodationsbedarfs in einem künstlichen Akkommodationssystem durch Messung der Augapfelorientierung unter Nutzung eines externen Magnetfelds. Das menschliche Auge ist ein optisches System, das mit Hilfe mehrerer lichtbrechender Grenzflächen Objekte scharf auf der Netzhaut (retina) abbildet. Hierbei passieren die Lichtwellen die Hornhaut (cornea), das Kammerwasser in der Vorderkammer (camera anterior bulbi), die Linse (lens crystallina) und den Glaskörper in der Hinterkammer (camera vitrea bulbi), die alle unterschiedliche Brechungsindizes aufweisen. Ändert sich die Gegenstandsweite des betrachteten Objektes, ist es für eine Abbildung mit gleich bleibender Schärfe auf der Netzhaut notwendig, dass sich das Abbildungsverhalten des optischen Systems ändert. Beim menschlichen Auge wird dies durch eine Verformung der Linse mit Hilfe des Ziliarmuskels (musculus ciliaris) realisiert, wodurch sich im Wesentlichen die Form und die Lage der Linsenvorder- und -rückseite ändern (Akkommodation). Bei einem intakten Akkommodationssystem eines jugendlichen Menschen kann so die Scheitelbrechkraft des Systems zwischen Ferneinstellung (desakkommodierter Zustand) und Naheinstellung (akkommodierter Zustand) um 14 dpt (Akkommodationsbreite) verändert werden. Dadurch können bei einem normalsichtigen (emmetropen) jugendlichen Menschen Objekte, die sich zwischen dem im Unendlichen liegenden Fernpunkt und dem sich in etwa 7 cm vor der Hornhaut liegenden Nahpunkt befinden, scharf auf der Netzhaut abgebildet werden. Da die Fähigkeit des menschlichen Auges zur Akkommodation mit zunehmendem Alter abnimmt, sind eine Anzahl von künstlich implantierbaren Linsensystemen mit variabler Brennweite entwickelt worden.

Bei potentiell akkommodierenden Intraokularlinsen handelt es sich um Linsen oder Linsensysteme, die nach operativer Entfernung der natürlichen Linse anstelle dieser eingesetzt und vorwiegend im Kapselsack befestigt werden. Durch eine noch vorhandene, jedoch geringe Restkontraktion des Ziliarmuskels, soll über eine Haptik eine axiale Verschiebung der Linse erreicht werden.

Beispiele für die Vielzahl der Entwicklungen sind unter anderem in DE 94 22 429 U1, DE 201 11 320 U1, DE 100 62 218 A1, DE 101 39 027, WO 02/083033, DE 101 25 829 A1, US 2004/0181279A1, US 2002/0149743, US 6 120 538, DE 101 55 345 C2, US 6 096 078, US 6 638 304, US 6 638 304, WO 00/4605 und WO 00/4605 beschrieben.

Vorrichtungen zur Wiederherstellung der Akkomodationsfähigkeit sind ferner aus der DE 101 55 345 C2, US 6 638 304 B2, WO 03/017873 A1 und US 43,73218 bekannt.

Ferner gibt es zahlreiche wissenschaftliche Veröffentlichungen zu dem Thema Akkommodationsfähigkeit von Linsensystemen. Beispielhaft sei auch auf folgende Veröffentlichung verwiesen:
Schneider, H.; Stachs, O.; Guthoff, R.: Evidenzbasierte Betrachtungen zu akkommodativen Kunstlinsen. 102. Jahrestagung der Deutschen Ophthalmologischen Gesellschaft (Berlin, Germany, September 23rd-26th 2004) (2004); Kammann, J.; Dornbach, G.: Empirical results regarding accommodative lenses. In: Current Aspects of Human Accommodation. Hrsg.: Guthoff, R.; Ludwig, K. Kaden Verlag Heidelberg (2001) 163-170, Fine, H.; Packer M.; Hoffmann R.: Technology generates IOL with amplitude of accommodation" (Ophtalmology Times Special Report, March 15th 2005) (2005), Lavin, M.: Multifocal intraocular lenses - part 1. Optometry Today 5/2001 (2001) 34-37; Lavin, M.: Multifocal intraocular lenses - part 2. Optometry Today 8/2001 (2001) 43-44; Nishi, O., Nishi, K.; Mano, C.; Ichihara, M.; Honda, T.: Controlling the capsular shape in lens refilling. Archives of Ophthalmology 115(4) (1997) 507-510; Fine, I.H.: The SmartLens- a fabulous new IOL technology. Eye World 7(10) (2002).

Das Problem der Akkommodation bis zu einem Leseabstand von ca. 30 cm wird durch den bisherigen Stand der Technik noch nicht zufriedenstellend gelöst. Grundsätzlich ist es nämlich durch die im Rahmen einer Kataraktextraktion implantierte Kunstlinse bisher nicht zufriedenstellend möglich, auf verschiedene Entfernungen zu fokussieren. Bisherige Versuche, intraokulare Strukturen, insbesondere die Ziliarmuskelaktivität zur mechanischen Brechkraftänderung implantierbarer Systeme zu nutzen, sind aus biologischen Gründen bisher nicht gelungen und dies ist auch mittelfristig nicht zu erwarten.

Aus dem Stand der Technik sind Lösungen zur intrakorporalen Bestimmung des Akkommodationsbedarfs über die Augapfelorierntierung des Augenpaares bekannt. Diese beschränken sich aber auf die Detektion der Kontraktion der äußeren Augenmuskeln, entweder durch Potentialmessung (z.B. US 6,638304) oder durch Messung der Andruckdifferenz zwischen Augapfel und zwei horizontalen Bulbusmuskeln an unterschiedlichen Augen (WO 2004/004605A1).

Die Erzeugung von starken, wechselnden elektromagnetischen Feldern sowie die Detektion der Bewegung kleiner Spulen setzen Geräte voraus, die aufgrund ihrer Masse und ihres Volumens für eine Implantation nicht geeignet sind. Die Messung der Kontraktion der Bulbusmuskeln über das Muskelpotential oder des Andrucks setzt eine Informationsverbindung zwischen Sensor und dem optisch aktiven Implantat im Kapselsack voraus. Eine Kabelverbindung würde den operativen Aufwand enorm erhöhen. Eine drahtlose Informationsübertragung setzt ein aktives System am Muskel voraus, welches ebenfalls über eine Energieversorgung verfügen müsste. Zusätzlich besteht bei der Verwendung von Elektroden das Problem von möglichen Gewebeänderungen im Elektrodenbereich. Daher sind beide Lösungen nicht praktikabel.

Externe Magnetfelder werden zur Bestimmung der Augapfelorientierung in einem Implantat noch nicht verwendet. Bisherige Lösungen zur Messung der Augapfelorientierung durch Magnetfelder bestehen nämlich aus Spulen in Kontaktlinsen, deren Ausrichtung im Raum durch die Wechselwirkung mit starken, zeitlich veränderlichen Magnetfeldern bestimmt werden kann.

Es ist Aufgabe der vorliegenden Erfindung, ein System vorzuschlagen, das in den Kapselsack implantierbar ist und seine Steuerimpulse unabhängig von der Ziliarkörperaktivität gewinnt.

Gelöst wird diese Aufgabe durch ein implantierbares System zur Bestimmung des Akkommodationsbedarfs in einem künstlichen Akkommodationssystem durch Messung der Augapfelorientierung beider Augen unter Nutzung eines externen Magnetfelds umfassend
a) wenigstens ein optisches System,
b) wenigstes ein zum Ziliarmuskel berührungsloses Informationserfassungssystem mit Mitteln zur Erfassung einer räumlichen Orientierung beider Augäpfel als körpereigenes Steuersignal für den Akkomodationsbedarf,
c) wenigstens ein Informationsverarbeitungssystem zwecks Erzeugung eines Stellsignals für das optische System aus den erfassten körpereigenen Steuersignalen
d) wenigstens ein Energieversorgungssystem und
e) wenigstens ein Befestigungssystem, wobei das System in beiden Augen je ein Mittel zur Messung eines Magnetfeldes aufweist und die Übertragungsmittel für den gegenseitigen Informationsaustausch zwischen den Mitteln vorgesehen sind.

Die einzelnen Subsysteme a) bis e) eines solchen einstückigen künstlichen Akkommodationssystems sind in der nicht vorveröffentlichten DE 102005038542 beschrieben. Diese Systeme sind zu einem, bzw. in mehreren Regelkreisen verschaltet. Das optische System, das Informationserfassungssystem, das Informationsverarbeitungssystem, das Energieversorgungssystem und das Befestigungssystem sind vorzugsweise zu einem Implantat zusammengefasst, welches zur Wiederherstellung der Akkommodationsfähigkeit des tierischen oder menschlichen Auges in dieses mittels des Befestigungssystems einsetzbar ist. Hierbei ist das optische System im Strahlengang des Auges angeordnet und bildet mit diesem zusammen den dioptrischen Apparat des Auges. In gleicher Weise sind vorzugsweise das Informationserfassungssystem, das Informationsverarbeitungssystem und das Energieversorgungssystem außerhalb des Strahlengangs angeordnet. Das Informationserfassungssystem kann sich auf mehrere Implantate (z. B. im linken und rechten Augapfel und im Oberkiefer) verteilen. Das Energieversorgungssystem kann vorzugsweise drahtlos mit einem externen System in Verbindung stehen. Die Schrift WO 2006/034336 beschreibt ein implantierbares System zur Bestimmung des Akkommodationsbedarfs in einem künstlichen Akkommodationssystem gemäss dem Obergriff des Anspruchs 1.

Das optische System, bestehend aus einem oder mehreren aktiv-optischen Elementen und / oder einer oder mehreren von Aktoren axial verschieblichen starren Linsen (=passiv-optisches Element), hat die Aufgabe, das Abbildungsverhalten im Strahlengang zu beeinflussen. Es muss im sichtbaren Wellenlängenbereich transparent sein und muss die Lage und / oder die Form mindestens einer seiner lichtbrechenden Grenzflächen zeitlich ändern können, um die Scheitelbrechkraft des dioptrischen Apparates zu verändern. Die aktorische Komponente besteht dabei aus Energiestellern und Energiewandlern (Grote / Feldhusen (Hrsg.): Dubbel - Taschenbuch für den Maschinenbau. 21. Auflage. Springer Verlag Berlin Heidelberg New York (2005)), welche unter Einwirkung von Stellsignalen einer informationsverarbeitenden Einrichtung Kräfte realisiert, die dann in Bewegung umgesetzt werden können.

Im Falle eines passiv-optischen Elementes werden eine oder mehrere starre Linsen von einem Aktor axial im Strahlengang verschoben. Dieses Wirkprinzip wird standardgemäß in technischen Produkten zur Fokussierung eingesetzt. DE4300840A1 beschreibt z.B. ein Varioobjektiv für Kompaktkameras, das aus zwei Linsengruppen besteht, deren Abstand relativ zueinander variiert werden kann, um eine Brennweitenverstellung durchzuführen.

Verschiedene Mechanismen können zur Erfüllung der oben beschriebenen Aufgabe eines aktiv-optischen Elements zum Einsatz kommen. Dabei ist zwischen einer Änderung der Brechungsindexverteilung und einer Krümmungsänderung einer zwei Medien unterschiedlicher Brechungsindizes trennenden Grenzfläche zu unterscheiden. Diese Veränderungen können durch verschiedene physikalische Wirkungsprinzipien realisiert werden, die im Folgenden dargestellt werden.

Brechungsindexänderung durch elektrooptische Materialien: Die doppelbrechende Eigenschaft elektrooptischer Materialien kann durch elektromagnetische Felder beeinflusst werden. Dadurch kann eine definierte Brechungsindexverteilung eingestellt werden, die eine gezielte Beeinflussung des Abbildungsverhaltens in einer Polarisationsebene des Lichts ermöglicht. Diese kann neben einer gezielten Änderung der Fokuslage auch die Korrektur von Abbildungsfehlern höherer Ordnung (z.B. Astigmatismen, sphärische Aberration, Koma) umfassen. Um beide zueinander senkrechten Polarisationsebenen gleichermaßen zu beeinflussen, ist eine rechtwinklig gekreuzte Hintereinanderanordnung zweier derartiger Systeme notwendig. In US6619799 wird die Verwendung eines derartigen aktiv-optischen Elementes in einem Brillengestell beschrieben. Dabei ist die elektrooptische Schicht von zwei transparenten Elektrodenflächen umfasst, zwischen denen eine elektrische Spannung angelegt werden kann, um das radiale Brechungsindexprofil zu verändern. Ein gewünschtes Brechungsindexprofil kann entweder durch Amplituden- und Frequenzmodulation der Steuerspannung oder durch Aufteilung der Elektroden in mehrere Bereiche, die mit jeweils unterschiedlichen Spannungen versorgt werden, erreicht werden.

Brechungsindexänderung durch Dichteänderung eines kompressiblen Fluids: Der Brechungsindex eines kompressiblen Fluids (z.B. eines Gases oder Gasgemisches) ist von der Dichte abhängig. Diese Abhängigkeit wird durch die Gladstone-Dale-Konstante beschrieben. Werden in einer gasgefüllten Kammer, die ein oder mehrere gekrümmte Begrenzungsflächen aufweist, der Druck und / oder die Temperatur geändert, ändert sich demnach auch das Abbildungsverhalten des optischen Systems. US4732458 beschreibt z.B. eine derartige Anordnung für ein Mehrlinsenelement, dessen Brechkraft kontinuierlich verändert werden kann. Die Druckerhöhung in der starren gasgefüllten Kammer wird durch einen in einem Zylinder geführten verschieblichen Kolben, der außerhalb der optischen Achse angeordnet ist, realisiert.

Geometrieänderung durch äußere Krafteinwirkung auf einen elastischen Festkörper: Ein elastischer Festkörper, dessen Brechungsindex sich von dem der Umgebung unterscheidet, kann durch Einwirkung äußerer Kräfte so verformt werden, dass sich die Krümmung seiner lichtbrechenden Oberflächen ändert und dadurch das optische Abbildungsverhalten beeinflusst wird. US6493151 beschreibt z.B. eine Anordnung für einen derartig verformbaren homogen oder inhomogen aufgebauten Festkörper, auf den durch einen in seinem Durchmesser veränderbaren Ring radiale Kräfte übertragen werden können. Die Durchmesseränderung des Rings kann thermisch, oder durch magnetische / elektrische Felder erfolgen. DE4345070 beschreibt beispielsweise eine Anordnung für einen verformbaren hüllenförmigen Festkörper, der mit einer lichtdurchlässigen Flüssigkeit gefüllt ist und dessen lichtbrechende Oberflächen über einen ringförmigen Fluidaktor hydraulisch oder pneumatisch verformt werden. DE10244312 nennt als Anwendungsbeispiel für einen Aktor aus Buckypaper (papierartiges Netzwerk von Kohlenstoffnanoröhren) die Änderung der Brechkraft einer künstlichen, in den Augapfel implantierten deformierbaren Linse.

Geometrieänderung durch Benetzungswinkelbeeinflussung (Electrowetting): Zwei ineinander nicht mischbare Fluide annähernd gleicher Dichte, die sich in ihren Brechungsindizes unterscheiden, bilden eine sphärisch gekrümmte oder plane Grenzfläche (Meniskus). Wird das eine, elektrisch leitfähige Fluid, in Kontakt mit einer Elektrode gebracht und gegenüber einer zweiten, von den beiden Fluiden durch eine isolierende Schicht (Dielektrikum) getrennte Elektrode eine Potentialdifferenz angelegt, so lässt sich der Benetzungswinkel und somit die Krümmung des Meniskus durch den sog. Elektrowetting-Effekt ändern. Da der Meniskus zwei Medien unterschiedlichen Brechungsindex trennt, wird das optische Abbildungsverhalten verändert. WO99/18456 beschreibt eine axiale Anordnung von leitfähigem Fluid, transparentem Dielektrikum und transparenter Elektrode im Strahlengang und Maßnahmen zur radialen Zentrierung des Tropfens in der optischen Achse. WO03/069380 beschreibt eine Anordnung, bei der die mit einem Dielektrikum beschichtete Elektrode zylindrisch um die optische Achse angeordnet ist. In der optischen Achse befinden sich axial hintereinander angeordnet das elektrisch-leitfähige Fluid und das isolierende Fluid, sowie der die beiden trennende Meniskus.

Geometrieänderung durch Druckänderung eines Fluides: Wird in einer fluidbefüllten Kammer, die eine oder mehrere deformierbare Begrenzungsflächen aufweist, die Druckdifferenz zur Umgebung geändert, kommt es zu einer Krümmungsänderung der Begrenzungsflächen und demnach auch zur Änderung des Abbildungsverhaltens des optischen Systems. US4466706 beschreibt beispielsweise eine derartige Anordnung, wobei die Druckdifferenzänderung durch einen Verdrängungsmechanismus erreicht wird. Dabei wird durch Drehung einer sich in der zylindrischen Ummantelung befindliche Schraube Fluid verdrängt, was zur Krümmungsänderung der beiden Zylinderendflächen führt. Alternativ kann die zylindrische Ummantelung auch zweiteilig ausgeführt sein, wobei eine Verdrängungswirkung durch eine axiale Relativbewegung beider Teile erzielt werden kann.

Geometrieänderung durch Kraftentwicklung innerhalb eines intelligenten Materials: Intelligente Materialien (Smart Materials) können durch Änderungen ihrer atomaren / molekularen Struktur Kräfte entwickeln und sich dadurch verformen. Durch die Einstellung eines Grenzflächenprofils zwischen intelligentem Material und Umgebung lässt sich demzufolge ebenfalls das optische Abbildungsverhalten beeinflussen. US2004/0100704 beschreibt z.B. einen zu diesem Zwecke verwendeten Formgedächtniskunststoff, der als Phase oder Schicht innerhalb eines verformbaren Linsenkörpers eingebracht ist und die Form des Körpers unter Einwirkung von Energie lokal verändern kann. Als Anwendungsbeispiel wird die postoperative, nicht reversible Korrektur des Abbildungsverhaltens implantierter Intraokularlinsen genannt. JP01230004 beschreibt beispielsweise die Verwendung eines schwellbaren Gels und eines Lösungsmittels, die innerhalb eines deformierbaren Festkörpers schichtartig angeordnet sind. Unter Einwirkung einer Spannung kann die Löslichkeit des Lösungsmittels im schwellbaren Gel so verändert werden, dass dieses daraufhin eine Volumenänderung erfährt. Diese bewirkt eine Krümmungsänderung der lichtbrechenden Oberfläche.

Auch Kombinationen der genannten Wirkprinzipien sind möglich. Das optische System ist folglich in der Lage, die Fokuslage des dioptrischen Apparates anzupassen. Das optische System kann des Weiteren mehrere Elemente umfassen, um das optische Abbildungsverhalten im Strahlengang zu optimieren. Ein enthaltenes aktiv-optisches Element kann gegebenenfalls weitere Abbildungsfehler (monochromatische und chromatische Aberrationen) statisch oder auch dynamisch korrigieren (lokale Beeinflussung der Lichtwellenfront).

Zur Generierung der Stellsignale für die aktorische Komponente des aktiv-optischen Elementes bzw. des passiv-optischen Elementes, ist es notwendig, Informationen zu erfassen, aus denen auf die notwendige Scheitelbrechkrafterhöhung (=Akkommodationsbedarf) geschlossen werden kann.

Informationen über den Akkommodationsbedarf können aus körpereigenen Steuersignalen der Okulomotorik gewonnen werden. Unter Steuersignalen werden hierbei Informationen verstanden, die den Sollwert oder den unter Einfluss des Sollwertes und möglicher Störsignale umgesetzten Istwert einer Regelstrecke enthalten. Für die Nutzung der Steuersignale der Okulomotorik, müssen Informationen beider Augen zusammen zur Ermittlung des notwenigen Akkommodationsbedarfs herangezogen werden.

Die Okulomotorik (vor allem die horizontale Vergenzbewegung) und der Akkommodationsbedarf sind beim Binokularsehen eindeutig miteinander gekoppelt. Die Fixierlinien beider Augen richten sich durch Rotation der Augäpfel so auf ein beliebig im Raum positioniertes Fixationsobjekt aus, dass das Bild des Fixationsobjektes auf korrespondierende Netzhautstellen fällt. Dadurch wird die Informationsfusion der beiden Einzelbilder zu einem Einfachbild im Gehirn ermöglicht. Die räumliche Orientierung der Augäpfel kann durch die Rotationen der Augäpfel um die drei Raumachsen beschrieben werden. Dabei treten bei jedem Augapfel separat betrachtet Rotationen um die horizontale Achse (Nickbewegung), um die mitgedrehte vertikale Achse (Gierbewegungen) und die mitgedrehte Fixierlinie (Rollbewegungen) auf. Dementsprechend können in Bezug auf beide Augen konjugierte Augenbewegungen (Versionen = gleichsinnige, gleichgroße Bewegung der Fixierlinien oder der Netzhautmeridiane beider Augen) und disjungierte Augenbewegungen

(Vergenzen = gegensinnige, gleichgroße Bewegung der Fixierlinien oder der Netzhautmeridiane beider Augen) unterschieden werden. Im Allgemeinen unterscheiden sich die Abstände des Fixationsobjektes zu den beiden mechanischen Augendrehpunkten und damit auch der Akkommodationsbedarf geringfügig (dies ist insbesondere dann der Fall, wenn sich das Fixationsobjekt nicht symmetrisch zu und nahe an den beiden Augen befindet). Die Erfassung der Steuersignale der Okulomotorik (Nervensignale oder Muskelsignale) ist extrakorporal möglich (z.B. durch die Elektromyographie der Augenmuskeln), sie wäre intrakorporal jedoch mit einem hohen Aufwand verbunden. Die Motorik der Augenbewegungen ist auch im hohen Alter hochpräzise und Abweichungen zwischen Soll- und Istwert (=Fixationsdisparität) betragen nur wenige Winkelminuten. Daher ist beim Binokularsehen in sehr guter Näherung ein Schnitt der Fixierlinien gewährleistet und es kann aus den Auswirkungen der Nerven- und Muskelsignale an die Augenmotorik, d.h. aus der Orientierung beider Augäpfel im Raum, auf den Akkommodationsbedarf des rechten, bzw. des linken Auges geschlossen werden.

Durch den Einsatz der Mittel zur Messung des Magnetfeldes lässt sich der Akkommodationsbedarf aus der Stellung der beiden Augäpfel berechnen. Durch den Schnittpunkt der Fixierlinien ist der Fixationspunkt eindeutig festgelegt. Der Kehrwert des Abstandes zwischen Hornhaut und Fixationspunkt entspricht dem Akkommodationsbedarf. Der Akkommodationsbedarf wird im wesentlichen durch die Augapfelorientierung, den Winkel, den die beiden Fixierlinien einschließen, bestimmbar. Liegt das Fixationsobjekt auf der Mittelsenkrechten der beiden Augenmitten, lässt sich der Akkommodationsbedarf aus dem Vergenzwinkel exakt berechnen. Liegt das Fixationsobjekt außerhalb der Mittelsenkrechten, ist diese Berechnung ausreichend genau.

Wird beispielsweise das Erdmagnetfeld als Referenz verwendet, lassen sich mit dem Mittel zur Messung des Magnetfeldes diejenigen Winkel bestimmen, die die Sensoren und damit auch die Augen bezüglich des Magnetfeldes einnehmen. Die Differenz der beiden Winkel entspricht dem Vergenzwinkel.

Bei einem kopffesten Magnetfeld lässt sich zusätzlich der Versionswinkel bestimmen. Dadurch ist in diesem Fall die Möglichkeit gegeben, bei einem Fixationspunkt außerhalb obengenannter Mittelsenkrechten den Unterschied des Akkommodationsbedarfs zwischen linkem und rechtem Auge zu bestimmen. Vorzugsweise sind erfindungsgemäß zwei augapfelfeste Mittel zur Messung eines Magnetfeldes vorgesehen. Die Augapfelorientierung lässt sich aufgrund dessen mit Hilfe eines externen Magnetfeldes, z.B. des Erdmagnetfeldes, oder auch eines anderen z.B. kopffesten Magnetfeldes messen. Als Mittel zur Messung eines Magnetfeldes können beispielsweise Magnetoresistiv- oder Hall-Sensoren vorgesehen werden. Diese sind beispielsweise aus zwei orthogonal zueinander stehenden Sensoren aufgebaut (Kompasssensor).

Hall-Sensoren und Magnetoresitiv-Sensoren wie auch Magnetfeldmessungen sind aus dem Stand der Technik im Allgemeinen bekannt.

Hall-Sensoren nutzen den Hall-Effekt zur Messung von Magnetfeldern und Strömen. Wird ein Hall-Sensor von einem Strom durchflossen und in ein senkrecht dazu verlaufendes Magnetfeld gebracht, liefert er eine Spannung, die proportional zum Produkt aus magnetischer Feldstärke und Strom ist. Ist der Strom bekannt, kann man die magnetische Feldstärke messen.

Der magnetoresistive Effekt beruht darauf, dass Magnetowiderstandseffekte den Widerstand von magnetischen bzw. nicht-magnetischen Metallen in Abhängigkeit von der Richtung (vektoriell) und den Betrag eines äußeren Magnetfeldes ändern.

Mit den erfindungsgemäß eingesetzten Magnetoresistiv- oder Hall-Sensoren ist es möglich, paarweise in je einem Auge ein Implantat zu schaffen. Vorzugsweise liegen die Größen der Sensoren im Bereich von 0,1 bis 4mm (Kantenlänge) und einer Dicke von 0,05 bis 1 mm. D. h. die bevorzugten Abmessungen der Sensoren liegen bei 5 mm x 5 mm x 2 mm, insbesondere 4 mm x 4 mm x 1 mm, ganz besonders bevorzugt bei 1 mm x 1 mm x 0,05 mm.

Alle Sensoren werden bevorzugt als gehäusefreie Chips in das System integriert. Auf diese Art sollen platzintensive Verdrahtungen im Gesamtsystem vermieden werden. Somit kann ermöglicht werden, dass das System am (z. B. in einer Kontaktlinse) oder im Augapfel appliziert wird.

Die Verwendung eines externen Magnetfeldes zur Messung der Augapfelorientierung ermöglicht eine ortsunabhängige Messung. Dadurch ist auch die Messung im Implantat im Kapselsack möglich und damit auch ein einstückiges Implantat erreichbar.

Die Möglichkeit für ein einstückiges künstliches Akkommodationssystem mit nur einem Implantationsort, dem Kapselsack, vereinfacht die Implantation erheblich. Da das System ohne elektrische oder taktile Anbindung an dem Körper messen kann, ist eine ausreichend genaue Messung unabhängig von eventuell auftretenden Gewebeveränderungen möglich. Dies gewährleistet ein dauerhaft funktionsfähiges System.

Da jedes Implantat für sich aus der Magnetfeldmessung seine Winkelstellung misst, der Akkommodationsbedarfs jedoch aus den Winkeln beider Augäpfel bestimmt werden muss, ist eine Informationsübertragung zwischen den beiden Implantaten notwendig. Für diese Informationsübertragung zwischen einem Implantat im linken und rechten Auge können beispielsweise elektromagnetische Wechselfelder verwendet werden. Werden elektromagnetische Wechselfelder verwendet, besteht die Möglichkeit, die vorhandenen Magnetsensoren als Informationsempfänger zu verwenden. Dies kann durch wechselseitiges Messen und Übertragen erfolgen oder auch durch die Wahl einer Trägerfrequenz, welche so hoch gewählt wird, dass keine Beeinflussung durch Störsignale auftritt, die durch Bewegungen von Kopf oder Augapfel hervorgerufen werden. Um die Funktion des Systems auch bei zeitlich beschränkt auftretenden Störungen des Erdmagnetfeldes sicherzustellen, können daraus entstehende Messfehler durch zusätzliche Sensoren zur Messung der Winkelbeschleunigung in beiden Augen (Gyroskop) kompensiert werden.

Die erfassten Informationen werden im Rahmen der hier beschriebenen Erfindung dem Informationsverarbeitungssystem zur Verfügung gestellt. Gegenstand der Erfindung ist aber auch ein oben beschriebenes Informationserfassungssystem allein, welches Messdaten zur Registrierung und Weiterverarbeitung an einen Empfänger außerhalb des Körpers senden kann.

Die erfassten Signale werden vom Informationsverarbeitungssystem aufbereitet (z.B. Ausreißertests, Glättung, Filterung, Verstärkung). Mit Methoden der klassischen Statistik, der Computational Intelligence und Data Mining werden Merkmale extrahiert und klassifiziert, um die Akkommodationsabsicht zu detektieren. Mit Hilfe von steuerungs- und regelungstechnischen Methoden (z.B. fuzzy-gesteuerter PID-Regler, adaptive Regelungsalgorithmen, selbstlernende Algorithmen) werden die benötigten Stellsignale für das optische System generiert. Es können sowohl hierarchische Regelungsstrukturen als auch zentral-dezentrale Strukturen zum Einsatz kommen.

Zur Versorgung der Subsysteme mit Energie wird ein Energieversorgungssystem eingesetzt, das aus einem Energiewandler, einem Energiespeicher und einer Steuerungseinheit bestehen kann. Der Energiewandler transformiert von außen fernübertragene Energie (z.B. induktiv, kapazitiv, optisch) oder gespeicherte Energie (z.B. Batterie, Miniatur-Brennstoffzelle), die auch in Form von Körperflüssigkeiten (z.B. das nährstoffreiche Kammerwasser, Blut) vorliegen kann, bzw. mechanische Energie (z.B. aus Muskelbewegungen) über einen Energiespeicher in elektrische Energie. Diese wird durch die Steuereinheit des Energieversorgungssystems zu genau definierten Zeitpunkten an die Subsysteme abgegeben. Durch Messung der Beleuchtungsstärke (z.B. durch eine Fotozelle) kann erreicht werden, dass bei Dunkelheit oder bei geschlossenen Augen, d.h. in Situationen, in denen keine Akkommodationsfähigkeit erforderlich ist, das Gesamtsystem in einen Zustand minimalen Energieumsatzes gebracht wird. Die dazu notwendigen Steuersignale werden vom Informationsverarbeitungssystem generiert.

Das Gesamtsystem wird mit Hilfe von geeigneten Befestigungselementen zur axialen Fixierung und radialen Zentrierung im Strahlengang implantiert. Aus der Ophthalmologie (Augenheilkunde) sind für Intraokularlinsen zahlreiche Haptikausführungen bekannt. (Draeger, J.; Guthoff, R.F.: Kunstlinsenimplantation. In: Augenheilkunde in Klinik und Praxis Band 4. Hrsg.: Francois, J.; Hollwich, F. Georg Thieme Verlag Stuttgart New York (1991); Auffarth, G.U.; Apple, D.J.: Zur Entwicklungsgeschichte der Intraokularlinsen. Ophthalmologe 98(11) (2001) 1017-1028). Diese können vorzugsweise im Kammerwinkel, im Sulcus ciliaris oder im Kapselsack Halt finden.

Das künstliche Akkommodationssystem ist der technische Teil eines Regelungssystems (geschlossener Regelkreis), welches als künstliches System die Funktion der natürlichen verformbaren Augenlinse und des Ziliarmuskels eines Patienten substituiert. Der biologische Teil besteht im Wesentlichen aus: der Hornhaut, dem Kammerwasser und dem Glaskörper als Bestandteile des dioptrischen Apparates, der Netzhaut als natürlichem Sensorarray und dem Gehirn als natürliche Informationsverarbeitungseinheit, die Steuersignale erzeugt, die Informationen über den Akkommodationsbedarf enthalten.

Das künstliche Akkommodationssystem umfasst ein optisches System, mit einer verstellbaren Brennweite und/oder anderen optischen Eigenschaften. Dieses bildet einen neu eingebrachten Bestandteil des dioptrischen Apparates des Patienten. Es umfasst ein Informationserfassungssystem, das Mittel zur Messung eines Magnetfeldes aufweist. Auf Basis dieser Messungen wird der Akkommodationsbedarf durch ein Informationsverarbeitungssystem ermittelt und es werden Stellsignale zum Ansteuern des optischen Systems generiert. Das System wird über ein geeignetes Energieversorgungssystem gespeist und ist über ein Befestigungssystem im Patientenauge fixiert.

Das beschriebene Akkommodationssystem kann zur Wiederherstellung der Akkommodationsfähigkeit nach Entfernung der natürlichen Augenlinse bei Linsentrübung (Katarakt) oder Alterssichtigkeit (Presbyopie) dienen.

Im Folgenden wird die Erfindung unter Bezugnahme auf die Figuren näher beschrieben:

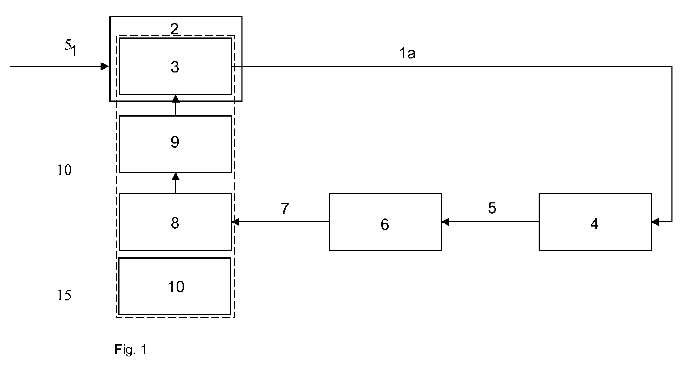

In Figur 1 ist eine schematische Darstellung des Gesamtsystems (künstliches Akkommodationssystem) wiedergegeben. Die Information 1, z.B. Licht von einem Objekt in zeitlich veränderlicher Gegenstandsweite fällt durch den dioptrischen Apparat des menschlichen Auges 2, der das optische System 3 enthält. Das fokussierte Licht 1 a fällt auf den natürlichen Sensor, die Netzhaut 4.

Die durch die Photorezeptoren generierten afferenten Signale 5 werden dem natürlichen Informationsverarbeitungssystem 6, dem Gehirn, zugeleitet. Von dort werden efferente Signale 7, die Information über den Akkommodationsbedarf enthalten, an motorische Strukturen (z.B. Ziliarmuskeln, Bulbusmuskeln) gesendet. Diese Information wird vom Informationserfassungssystem 8 des künstlichen Akkommodations-Systems aufgenommen. Das Informationsverarbeitungssystem 9 leitet daraus Stellsignale für das optische System 3 ab. Damit wird die Scheitelbrechkraft des dioptrischen Apparates 2 durch das künstliche Akkommodationssystem an den Akkommodationsbedarf angepaßt, der aus zeitlich veränderlichen Gegenstandsweiten resultiert. 10 stellt das Energieversorgungssystem dar. Alle technischen Systembestandteile sind durch eine gestrichelte Linie eingerahmt.

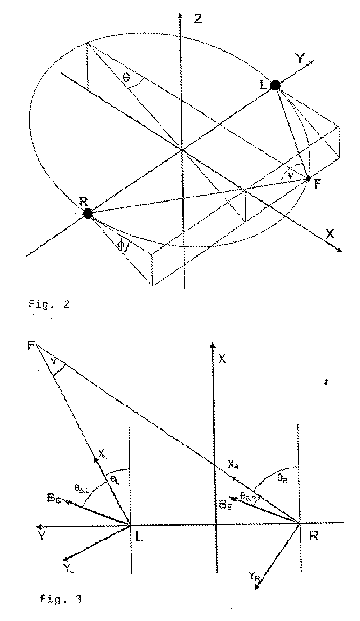

In Figur 2 sind der Vergenzwinkel v, der Versionswinkel θ und der Knickwinkel Φ bei Betrachtung eines Fixationswinkels F dargestellt. Damit lässt sich der Akkommodationsbedarf aus der Stellung der beiden Augäpfel berechnen. Durch den Schnittpunkt der beiden Fixierlinien ist der Fixationspunkt des Fixationsobjektes F vorgegeben. Der Kehrwert des Abstandes zwischen Hornhaut und Fixationsobjekt F entspricht dem Akkommodationsbedarf. Der Akkommodationsbedarf wird im Wesentlichen durch den Vergenzwinkel v und den Versionswinkel θ bestimmbar. Liegt das Fixationsobjekt auf der Mittellinie der beiden Augenmitten, lässt sich der Akkommodationsbedarf aus dem Vergenzwinkel v exakt berechnen.

In Figur 3 ist die Augenstellung bezüglich des annähernd homogenen Erdmagnetfeldes dargestellt. Mit Hilfe dieses Erdmagnetfeldes kann der Vergenzwinkel v unter Einbeziehung von zwei augäpfelfesten Kompasssensoren gemessen werden. Durch die Verwendung des Erdmagnetfeldes als Referenz lassen sich mit den beiden Kompasssensoren diejenigen Winkel bestimmen, die die Sensoren und damit die Augen bezüglich des Magnetfeldes einnehmen. Die Differenz der beiden Winkel entspricht dem Vergenzwinkel v.

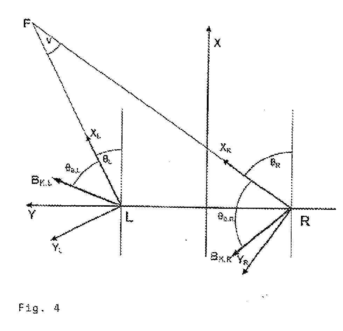

In Figur 4 ist die Augenstellung bezüglich des beliebigen, aber kopffesten Magnetfeldes dargestellt. Bei einem kopffesten Magnetfeld lässt sich nämlich zusätzlich zum Vergenzwinkel v der Versionswinkel θ erfassen. Dadurch ist in jedem Fall die Möglichkeit gegeben, bei einem Fixationsobjekt F außerhalb der Mittelsenkrechten den Unterschied des Akkommodationsbedarfs zwischen linken und rechten Auge zu bestimmen.

## Patentansprüche

1. Implantierbares System zur Bestimmung des Akkommodationsbedarfs in einem künstlichen Akkommodationssystem durch Messung der Augapfelorientierung unter Nutzung eines externen Magnetfeldes umfassend
a) wenigstens ein optisches System (3),
b) wenigstens ein zum Ziliarmuskel berührungsloses Informationserfassungssystem (8) mit Mitteln zur Erfassung einer räumlichen Orientierung beider Augäpfel als körpereigenes Steuersignal für den Akkommodationsbedarf,
c) wenigstens ein Informationsverarbeitungssystem (9) zwecks Erzeugung eines Stellsignals für das optische System (3) aus den erfassten körpereigenen Steuersignalen
d) wenigstens ein Energieversorgungssystem (10) und
e) ein Befestigungssystem,
**dadurch gekennzeichnet, dass** das System in beiden Augen je ein Mittel zur Messung des externen Magnetfeldes aufweist und Übertragungsmittel für den gegenseitigen Informationsaustausch zwischen den Mitteln vorgesehen sind.

2. System nach Anspruch 1,
**dadurch gekennzeichnet, dass** die Mittel zur Messung des externen Magnetfeldes jeweils fest mit dem jeweilig zugehörigen Augapfel verbunden sind oder in diesen eingesetzt sind.

3. System nach Anspruch 1 oder 2,
**dadurch gekennzeichnet, dass** das Magnetfeld durch ein Erdmagnetfeld gebildet wird.

4. System nach Anspruch 1 oder 2,
**dadurch gekennzeichnet, dass** das Magnetfeld durch ein kopffestes Magnetfeld gebildet wird.

5. System nach einem der vorgenannten Ansprüche,
**dadurch gekennzeichnet, dass** die Mittel zur Messung des Magnetfeldes jeweils einen Kompasssensor oder jeweils zwei in ihrer Messrichtung eine Ebene aufspannende Magnetfeldsensoren umfassen.

6. System nach Anspruch 5,
**dadurch gekennzeichnet, dass** die Kompasssensoren bzw. die Ebenen in beiden Augen parallel zu einer Gerade, die die Augendrehpunkte beider Augen verbindet, und parallel zueinander ausgerichtet sind.

7. System nach Anspruch 5 oder 6,
**dadurch gekennzeichnet, dass** die Mittel zur Messung des Magnetfeldes durch Magnetoresistiv oder Hall-Sensoren gebildet sind.

8. System nach einem der vorgenannten Ansprüche,
**dadurch gekennzeichnet, dass** die Messmittel als Übertragungsmittel und damit als Informationsempfänger für Wechselfelder und Signale einsetzbar sind.

## Claims

1. An implantable system for determining the accommodation requirement in an artificial accommodation system by measuring the eyeball orientation using an external magnetic field, comprising
a) at least one optical system (3),
b) at least one data acquisition system (8) which does not contact the ciliary muscle and has means for measuring a spatial orientation of both eyeballs as a physical control signal for the accommodation requirement,
c) at least one data processing system (9) for generating an actuating signal for the optical system (3) from the measured physical control signals,
d) at least one energy supply system (10), and
e) one fixing system,
**characterized in that** the system in each case has means for measuring the external magnetic field in both eyes and provision is made for transfer means for mutual information exchange between the means.

2. The system as claimed in claim 1,
**characterized in that** the means for measuring the external magnetic field are in each case fixedly connected to the respectively associated eyeball or are inserted therein.

3. The system as claimed in claim 1 or 2,
**characterized in that** the magnetic field is formed by a terrestrial magnetic field.

4. The system as claimed in claim 1 or 2,
**characterized in that** the magnetic field is formed by a magnetic field that is fixed with respect to the head.

5. The system as claimed in one of the preceding claims, **characterized in that** the means for measuring the magnetic field respectively comprise a compass sensor or two magnetic field sensors which span a plane with their measurement directions.

6. The system as claimed in claim 5,
**characterized in that** the compass sensors or the planes in both eyes are aligned parallel with respect to a straight line, which connects the pivotal points of both eyes, and are mutually parallel.

7. The system as claimed in claim 5 or 6,
**characterized in that** the means for measuring the magnetic field are formed by magnetoresistive or Hall sensors.

8. The system as claimed in one of the preceding claims, **characterized in that** the measurement means can be used as transfer means and hence as data receivers for alternating fields and signals.

## Revendications

1. Système implantable pour déterminer le besoin en accommodation dans un système d'accommodation artificiel en mesurant l'orientation du globe oculaire en utilisant un champ magnétique externe, comprenant
a) au moins un système optique (3),
b) au moins un système d'acquisition d'informations (8) sans contact à propos du muscle ciliaire, pourvu de moyens destinés à détecter une orientation spatiale des deux globes oculaires en tant que signal de commande propre au corps pour le besoin d'accommodation,
c) au moins un système de traitement d'informations (9) servant à générer un signal de réglage pour le système optique (3) à partir des signaux de commande propres au corps détectés,
d) au moins un système d'alimentation en énergie (10) et
e) un système de fixation,
**caractérisé en ce que** le système possède dans les deux yeux à chaque fois un moyen de mesure du champ magnétique externe et il existe des moyens de transmission pour l'échange mutuel d'informations entre les moyens.

2. Système selon la revendication 1, **caractérisé en ce que** les moyens de mesure du champ magnétique externe sont respectivement reliés à demeure avec le globe oculaire associé correspondant ou sont logés dans celui-ci.

3. Système selon la revendication 1 ou 2, **caractérisé en ce que** le champ magnétique est formé par un champ magnétique terrestre.

4. Système selon la revendication 1 ou 2, **caractérisé en ce que** le champ magnétique est formé par un champ magnétique fixe sur la tête.

5. Système selon l'une des revendications précédentes, **caractérisé en ce que** les moyens de mesure du champ magnétique comprennent respectivement un capteur de compas ou respectivement deux capteurs de champ magnétique dont la direction de mesure couvre un plan.

6. Système selon la revendication 5, **caractérisé en ce que** les capteurs de compas ou les plans dans les deux yeux sont orientés parallèlement à une droite qui relie les points de rotation des deux yeux et parallèlement l'un à l'autre.

7. Système selon la revendication 5 ou 6, **caractérisé en ce que** les moyens de mesure du champ magnétique sont formés par des capteurs magnétorésistifs ou à effet Hall.

8. Système selon l'une des revendications précédentes, **caractérisé en ce que** les moyens de mesure sont utilisables en tant que moyens de transmission et ainsi comme récepteurs d'informations pour les champs alternés et les signaux.
